# EUROPEAN PATENT SPECIFICATION

(11) **EP 3 317 672 B1**
(45) Date of publication and mention of the grant of the patent: **11.12.2019**
(21) Application number: 16736595.6
(22) Date of filing: 01.07.2016
(51) Int. Cl.: G01N 33/574, G01N 33/68, C12Q 1/68

(54) **USE OF CELL-FREE NUCLEOSOMES AS BIOMARKERS IN FECAL SAMPLES**
VERWENDUNG VON ZELLFREIEN NUKLEOSOMEN ALS BIOMARKER IN FÄKALPROBEN
UTILISATION DE NUCLÉOSOMES ACELLULAIRES COMME BIOMARQUEURS DANS DES ÉCHANTILLONS DE MATIÈRES FÉCALES

(30) Priority: 01.07.2015 GB 201511542
(43) Date of publication of application: 09.05.2018
(73) Proprietor: Belgian Volition SPRL, 5032 Isnes (BE)
(72) Inventor: MICALLEF, Jacob, BE-5032 Isnes (BE); HERZOG, Marielle, BE-5032 Isnes (BE)
(74) Representative: Pond, Elizabeth Grace Catherine
(86) International application number: PCT/GB2016/051995
(87) International publication number: WO 2017/001864

(56) References cited:
- WO-A1-2013/030577
- WO-A1-2013/030579
- WO-A1-2014/131841
- WO-A2-2013/084002
- GIULIA DE MAIO: "Circulating and stool nucleic acid analysis for colorectal cancer diagnosis", WORLD JOURNAL OF GASTROENTEROLOGY, vol. 20, no. 4, 28 January 2014 (2014-01-28), page 957, XP055299064, CN ISSN: 1007-9327, DOI: 10.3748/wjg.v20.i4.957

## Description

### FIELD OF THE INVENTION

The invention relates to the use of cell-free nucleosomes, and in particular an epigenetic feature of cell-free nucleosomes as a biomarker for disease. The invention also provides a method for the detection of colorectal cancer and polyps by detecting and measuring the presence of nucleosomes and epigenetically altered nucleosomes in fecal samples. In particular the method relates to the detection and epigenetic profiling of nucleosome chromatin fragments in fecal samples and using this epigenetic information to establish their cellular origination in the chromatin of healthy colorectal cells or in the chromatin of colorectal cancer or colorectal adenoma cells.

### BACKGROUND OF THE INVENTION

Colorectal Cancer (CRC) is a common disease with a high mortality. The biology of the disease is understood to involve a progression from pre-cancerous adenoma (polyp) with increasing dysplasia leading to stage I, II, III and eventually stage IV CRC. Mortality varies greatly depending on whether the disease is detected at an early localized stage, when effective treatment options are available, or at a late stage when the disease may have spread within the colon or rectum or beyond when treatment is more difficult. The 5-year survival rate is >90% for those in whom the disease is detected at stage I, but only about 10% for those in whom stage IV metastatic disease is detected. For this reason many countries have CRC screening programmes to identify individuals with CRC or precancerous adenomas or polyps. CRC incidence is age and sex dependent; being more common in men than women and more common in elderly people. CRC is rare in people below the age of 50 so it is more useful to screen older people. The actual screening age range tested varies in screening programmes in different countries but is typically of the order of 50-74 years.

CRC screening programmes enable earlier CRC detection than would otherwise be the case, leading to earlier treatment and many years of saved life. Earlier CRC detection also saves money and resources for healthcare providers by reducing the need for expensive late stage cancer drug therapies and hospitalizations. Ideally CRC screening programmes would also detect precancerous colorectal polyps or adenomas which may progress to CRC if left untreated, but can be removed before cancer develops if detected. The prognosis for such patients is good.

The methods commonly used for CRC detection and/or screening all suffer from major drawbacks. The primary CRC screening method employed in the USA is colonoscopy. Colonoscopy essentially involves visually examining the colon using a scope which traverses the descending, transverse and ascending colon to find any cancerous or potentially pre-cancerous lesions. The primary advantage of colonoscopy is its accuracy of detection which is of the order of 95% clinical sensitivity for CRC as well as very high detection rates for precancerous adenomas all with very high clinical specificity (>95%). This accuracy makes colonoscopy the gold standard for CRC detection, especially as low grade lesions can be removed at the time of their detection. However, colonoscopy suffers from a number of limitations as a frontline CRC detection or screening method. Colonoscopy is a highly invasive procedure requiring a surgical admission, the procedure is usually performed under anesthesia, requires preparation of the bowel by the patient in advance, causes injury to the patient in some cases (for example tearing of the bowel) and is expensive (>$1000). CRC is detected in approximately 0.5% of screening colonoscopies so the vast majority of people screened are subjected to a surgical procedure for little benefit. Due to these disadvantages, patient compliance with colonoscopy is low and many people of screening age do not undergo the procedure. For these reasons, colonoscopy is not used as a frontline CRC detection or screening method in most countries of the world.

Some healthcare providers employ a related method called sigmoidoscopy in which a shorter scope is used to examine the descending colon only. Although this method misses two thirds of the colon, it does examine the area where cancers are most commonly observed. The disadvantages of sigmoidoscopy are similar to those of colonoscopy and it is not commonly used as a frontline test for similar reasons. Virtual colonoscopy, or computerized tomography (CT) colonography, is also used. This procedure employs a combination of x-rays and computer technology to create images of the rectum and colon to detect colorectal tumors and polyps.

The most commonly used CRC detection and screening methods involve a two stage procedure in which the population of screening age is first screened with a non-invasive frontline fecal test to identify a subgroup of the screening population in whom there is a higher risk of CRC. People who test positive in the fecal test are referred for a follow-up colonoscopy. About 5% of these people will typically be found to have CRC. The result of fecal screening is negative for the majority of people, so the two stage method prevents unnecessary colonoscopies on most people with no lesion. However, current fecal tests have poor clinical accuracy and there is a need for affordable, more accurate fecal CRC tests.

The principle underlying current fecal tests for CRC is the detection of bleeding into the colon or rectum. In simple terms; when the colon or rectum is partially blocked by an intruding cancerous or precancerous growth, movement of the stool past the blockage is likely to cause injury and bleeding. This bleeding is detected by testing the fecal sample for the presence of hemoglobin. As the degree of bleeding may vary greatly from day to day, the test may need to be performed several times on separate days.

All fecal CRC tests are designed to detect fecal hemoglobin and these can be divided into three main types. The guaiac fecal occult blood test (FOBT or gFOBT) is a chemical test method for hemoglobin in which the patient or operator typically smears a small amount of feces on to an alpha-guaiaconic acid coated paper or other substrate. If blood is present in the feces, addition of hydrogen peroxide to the paper produces a rapid color change through the oxidation of alpha-guaiaconic acid to a blue colored quinone in a reaction catalyzed by heme (a component of hemoglobin). The consumption of meat (and hence heme) as well as some vegetables, that contain other catalyst molecules that behave like heme in the test, can cause false positive results. Similarly some substances, including vitamin C can lead to false negative results so dietary restriction is often advised prior to the test. Guaiac FOBT tests can have high clinical specificity depending on the cut-off used and have 60-70% sensitivity for detection of CRC. Detection of precancerous polyps or adenomas is poor. Chemical FOBT methods were the method of choice in the past and, although still widely used, are being displaced by fecal immunochemical test methods.

Fecal immunochemical test, or FIT, methods (also called iFOBT or FOBTi) are essentially immunoassay tests for human hemoglobin in fecal samples. FIT methods are less susceptible to false positive and negative results due to interference of dietary factors and can detect smaller amounts of blood in the feces. These tests have similar specificity to gFOBT but detect slightly more clinically relevant cancer lesions. Detection of polyps or adenomas is poor.

The third type of fecal test for CRC detection is a more recent innovation and involves analysis of DNA sequences in the stool in addition to analysis for hemoglobin. In these methods DNA is extracted from the stool and analyzed to identify cancer associated sequence mutations and/or gene specific DNA methylation changes. An example of this approach is provided by the Cologuard test produced by Exact Sciences Corporation. The test involves a panel of 11 markers for CRC in the stool including hemoglobin, 7 DNA sequence mutations, 2 DNA sequence methylations and the DNA sequence for beta actin (as a control for normalization of results). In this test, the patient stool sample is processed at the laboratory to isolate the DNA from the stool. The DNA is then amplified and NDRG4 and BMP3 gene methylation is investigated by quantitative allele-specific real-time target and signal amplification. Cancer associated KRAS mutations are also investigated using these assays and all are assessed in relation to the level of total DNA assessed by beta actin amplification. DNA methylation, mutation and hemoglobin results are combined to provide a positive or negative result. The Cologuard test is more accurate than simple FOBT or FIT tests and detects 92% of cancers and 42% of polyps with a specificity of 87% (The Medical Letter, 2014).

Most FOBT and FIT fecal screening programmes report low (50-60%) patient uptake. This is usually assumed to be due to the distasteful requirement for handling of fecal material in addition to the low accuracy of the tests. The Cologuard fecal test has a higher accuracy but whether this will increase patient compliance remains to be established as it has only recently been approved for use in the USA (August 2014), and is not yet in common use. The main disadvantage of the Cologuard method is its high cost due to the complex nature of its panel test involving multiple assay methods performed on multiple assay platforms including immunoassay as well as both specific DNA sequence analysis and DNA sequence methylation analysis. The high cost is likely to rule out widespread screening use in most countries. The complexity and multi-platform nature of the test also means that it is unlikely to be performed outside of sophisticated dedicated laboratories.

Classical biomarkers including carcinoembryonic antigen (CEA) have been investigated as possible blood based biomarkers for CRC but their clinical accuracy is too low for routine use and they are better used for patient monitoring. More recently, hypermethylation of specific gene sequences have been investigated for use as diagnostic biomarkers for cancers in blood. For example a method reported for detection of hypermethylation of the Septin 9 gene by PCR amplification of DNA extracted from plasma was reported to detect 72% of colon cancers with a false positive rate of 10% (Grutzmann et al, 2008). The DNA methylation status of specific genes or loci is usually detected by selective bisulphite deamination of cytosine, but not 5-methylcytosine, to uracil, leading to a primary DNA sequence change that can be detected by sequencing or other means (Allen et al, 2004).

There is a clinical need for accurate, safe, non-invasive, low cost tests to identify individuals with early stage colorectal cancer or precancerous polyps. Unfortunately, all tests currently available are either inaccurate, invasive or high cost.

The present invention relates to the detection of cell-free nucleosomes and epigenetic features of cell-free nucleosomes in fecal samples. Cell-free nucleosomes have previously been reported in the blood where they are thought to originate from dead or dying cells. The mechanism of release from dead cells into the circulation is not known but is thought to involve circulating macrophages that remove dead cells by phagocytosis (Jiang et al, 2003). The level of circulating nucleosomes found in the blood of healthy subjects is low, but elevated levels are often found in the blood of subjects with a variety of disease conditions including CRC cancer (Holdenrieder, 2001). However, elevated blood nucleosome levels are a non-specific marker of elevated levels of cell death and do not distinguish CRC from other cancers or from other diseases or from injuries or trauma. There are no reports of nucleosomes in the feces.

Nucleosomes have an immense diversity of chemical structure due to their role in the epigenetic regulation of gene expression. The human body comprises many different cell types. All of these cell types contain the same genome but widely different phenotypes and different functions in the body. This phenotypic diversity is due to the differential expression of the genome in different cell types. The control of differential gene expression is not entirely understood but the basic mechanisms include gene regulation by a number of interconnected epigenetic signals associated with the gene, including non-coding nucleic acid sequences as well as structural chromatin aspects including control of chromatin packing (for example; as euchromatin or heterochromatin), control of nucleosome positioning and nuclease accessible sites, chemical modification of DNA, for example methylation of cytosine residues, and variation in the composition and structure of the histone complex around which the DNA is wrapped, for example by post-translational modification of histone proteins or by inclusion of alternative histone isoforms or variants (different histone gene products). Thus cellular chromatin, and its constituent nucleosomes, comprise a great variety of chemical structural epigenetic features which are subject to a variety of alterations which together form a complex gene regulatory system. This regulatory system is corrupted in cancer leading to global epigenomic changes in chromosome structure. The altered chromatin structure leads to gene mis-regulation which is associated with cancer development and progression. Moreover, prevention or reversal of these epigenetic chemical feature changes to chromatin and nucleosomes using epigenetic drugs is effective in the treatment of cancer diseases. Such epigenetic drugs include, for example, Histone Deacetylase Complex inhibitor (HDACi), Histone Methyl Transferase inhibitor (HMTi) and DNA Methyl Transferase inhibitor (DNMTi) drugs.

The nucleosome is the basic repetitive unit of chromatin structure and consists of a protein complex of eight highly conserved core histones (comprising of a pair of each of the histones H2A, H2B, H3, and H4). Around this complex is wrapped approximately 146 base pairs of DNA. Another histone, H1 or H5, acts as a linker and is involved in chromatin compaction. The DNA is wound around consecutive nucleosomes in a structure often said to resemble "beads on a string" and this forms the basic structure of open or euchromatin. In compacted or heterochromatin this string is coiled and super coiled into a closed and complex structure (Herranz and Esteller, 2007).

The structure of nucleosomes in cellular chromatin can vary by Post Translational Modification (PTM) of histone proteins and by the inclusion of variant histone proteins. PTM of histone proteins occurs most commonly, but not only, on the tails of the eight core histones and common modifications include acetylation, methylation or ubiquitination of lysine residues as well as methylation of arginine residues and phosphorylation of serine residues. Histone modifications are known to be involved in epigenetic regulation of gene expression (Herranz and Esteller, 2007). The structure of the nucleosome can also vary by the inclusion of alternative histone isoforms or variants which are different gene or splice products and have different amino acid sequences. Histone variants can be classed into a number of families which are subdivided into individual types. The nucleotide sequences of a large number of histone variants are known and publicly available for example in the National Human Genome Research Institute NHGRI Histone DataBase (http://genome.nhgri.nih.gov/histones/complete.shtml), the GenBank (NIH genetic sequence) DataBase, the EMBL Nucleotide Sequence Database and the DNA Data Bank of Japan (DDBJ).

Histone variant and histone modification patterns present in healthy and diseased cells have been shown to differ in numerous (mostly immunohistochemical) studies (Herranz and Esteller, 2007). One disadvantage of immunohistochemical methods for clinical use is that tissue sample collection is invasive involving surgery or biopsy.

Nucleosome structure also varies by chemical modification of their DNA component, including the methylation status of DNA (Herranz and Esteller, 2007). It has been known in the art for some time that DNA may be methylated at the 5 position of cytosine nucleotides to form 5-methylcytosine and the involvement of DNA methylation in cancer was reported as early as 1983 (Feinberg and Vogelstein, 1983). DNA methylation patterns observed in cancer cells differ from those of healthy cells. Repetitive elements, particularly around pericentromeric areas, are reported to be hypomethylated in cancer relative to healthy cells but promoters of specific genes are reported to be hypermethylated in cancer. The balance of these two effects is reported to result in global DNA hypomethylation in cancer and this is a hallmark of cancer cells (Esteller 2007, Hervouet et al, 2010, Rodriguez-Paredes & Esteller, 2011).

Nucleosome structure also varies by nucleosome binding to any of a multitude of other proteins present in chromatin to form nucleosome adducts. Chromatin comprises a large number of non-histone proteins of a wide variety of types with a variety of functions including transcription factors, transcription enhancement factors, transcription repression factors, histone modifying enzymes, DNA damage repair proteins, nuclear hormone receptors and many more. The study of chromatin bound proteins has been carried out largely by Chromatin ImmunoPrecipitation (ChIP) methods. These methods are well known in the art but are complex, laborious and expensive.

Cell free nucleosomes *per se* can be detected by Enzyme-Linked ImmunoSorbant Assay (ELISA) and several methods have been reported (Salgame et al, 1997; Holdenrieder et al, 2001; van Nieuwenhuijze et al, 2003). These assays typically employ an anti-histone antibody (for example anti-H2B, anti-H3 or anti-H1, H2A, H2B, H3 and H4) as capture antibody and an anti-DNA or anti-H2A-H2B-DNA complex antibody as detection antibody.

We have previously reported ELISA assays for nucleosomes containing particular epigenetic signals including nucleosomes containing particular histone modifications, particular histone variants and particular DNA modifications as well as particular nucleosome adducts (WO 2005/019826, WO 2013/030579, WO 2013/030577, WO 2013/084002). We have previously used these assays to show that epigenetically altered circulating cell free nucleosomes can be detected in the blood of diseased patients.

Surprisingly we have now demonstrated the detection of cell free nucleosome chromatin fragments in fecal samples obtained from patients and have shown that the epigenetic nature or structural profile of these nucleosomes can be used to determine their origination. Chromatin fragments have been identified as originating from healthy cells or from diseased gastroenterological cells including from colorectal cancer cells and polyp cells or from a mixture of these cells. Unlike previous fecal methods such as the Cologuard method, the present invention involves no DNA sequencing of particular genes but relies on measurement of fecal nucleosome levels *per se* as well as the investigation of genome wide epigenetic alteration of nucleosome or other chromatin fragments in the feces. We now report simple immunoassay methods for the direct estimation of nucleosomes and epigenetically altered cell free nucleosomes and nucleosome adducts in fecal samples and their determination as having a healthy, CRC or polyp cell chromatin origin.

### SUMMARY OF THE INVENTION

According to a first aspect of the invention there is provided the use of a cell-free nucleosome as a biomarker in a fecal sample for the diagnosis or detection of colorectal cancer, a colorectal adenoma or a polyp.

According to a second aspect of the invention there is provided a method for diagnosing or detecting colorectal cancer, a colorectal adenoma or a polyp in an animal or a human subject which comprises the steps of:(i) detecting or measuring an epigenetic feature of a cell-free nucleosome in a fecal sample obtained from the subject; and(ii) using the measured level of cell-free nucleosomes containing said epigenetic feature as indicative of the presence of said disease in the subject.

### BRIEF DESCRIPTION OF THE FIGURES

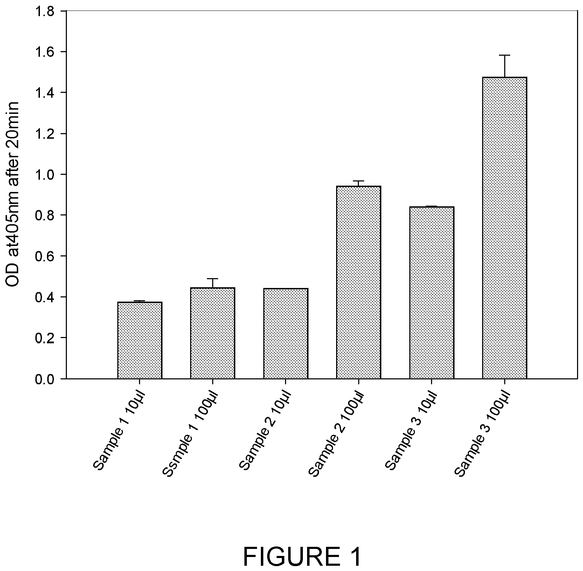

Figure 1. ELISA results for the detection of cell free nucleosomes in diluted fecal samples taken from 3 subjects.

### DETAILED DESCRIPTION OF THE INVENTION

The invention relates to the use of cell-free nucleosomes in fecal samples, and in particular an epigenetic feature of cell-free nucleosomes as a biomarker for disease. The invention also provides a method for the detection of colorectal cancer and polyps by detecting and measuring the presence of nucleosomes and epigenetically altered nucleosomes in fecal samples. In particular, the method relates to the detection and epigenetic profiling of nucleosome chromatin fragments in fecal samples and using this epigenetic information to establish their cellular origination in the chromatin of healthy colorectal cells or in the chromatin of colorectal cancer or colorectal adenoma cells. We have previously reported the detection and epigenetic profiling of nucleosome chromatin fragments in blood samples (WO 2005/019826, WO 2013/030579, WO 2013/030577, WO 2013/084002). The presence of altered epigenetic chromatin fragments in a blood sample taken from a subject may be used to indicate the presence of a cancer in that subject, but may be less informative of the particular organ affected by that cancer because all tissues are perfused by blood. The particular advantage of the use of fecal samples is that the sample itself limits the origin of altered epigenetic chromatin fragments to the gastrointestinal tract. Furthermore, as nucleosomes originating in the upper areas of the tract are likely to be digested, the presence of altered epigenetic chromatin fragments in a fecal sample taken from a subject may be used to indicate the presence of a cancer in that subject and identify that cancer as a colorectal cancer or precancerous polyp.

According to a first aspect of the invention there is provided the use of a cell-free nucleosome as a biomarker in a fecal sample for the diagnosis or detection of colorectal cancer, a colorectal adenoma or a polyp. We have shown that the level of nucleosomes (*per se*) is altered in the stool samples of diseased patients relative to the levels of stool samples of healthy subjects and that these nucleosomes comprise different absolute and relative levels of a variety of epigenetic structures. One advantage of feces as a patient sample is the degree of organ specificity its use confers on biomarker tests.

In one embodiment, the biomarker is used for the diagnosis or detection of colorectal cancer or colorectal polyps.

In one embodiment, the biomarker comprises the level of nucleosomes *per se.* It will be appreciated that the level of fecal nucleosomes *per se* may be estimated, for example using immunoassay methods for nucleosomes similar to those known in the art (Salgame et al, 1997; Holdenrieder et al, 2001; van Nieuwenhuijze et al, 2003).

In one embodiment, the biomarker comprises an epigenetically altered or otherwise modified cell free nucleosome. Thus, for example, the biomarker is an epigenetic feature of a cell-free nucleosome.

In one embodiment, the cell-free nucleosome is a mononucleosome, oligonucleosome or other chromosome fragment.

According to a second aspect of the invention there is provided a method for diagnosing or detecting colorectal cancer, a colorectal adenoma or a polyp in an animal or a human subject which comprises the steps of:(i) detecting or measuring an epigenetic feature of a cell-free nucleosome in a fecal sample obtained from the subject; and(ii) using the measured level of cell-free nucleosomes containing said epigenetic feature as indicative of the presence of said disease in the subject.

In one embodiment, the epigenetic feature is selected from: a post-translational histone modification, a histone variant or isoform, a DNA modification or a protein adducted to the nucleosome

In a further embodiment, the epigenetic feature of a cell-free nucleosome comprises one or more histone post-translational modifications. A large number of such histone modifications are known in the art and this number is increasing with newly identified modifications. For example, without limitation, histones contain a variety of amino acid residues at multiple positions including lysine, serine, arginine and threonine residues in histones H2, H3 and H4, as well as their isoforms or sequence variants. These may be modified in multiple ways including, for example without limitation, acetylation, ubiquitination, biotinylation or mono, di, or tri-methylation of lysine residues. Any histone modification may be a suitable feature for use in the invention whether detected or measured as an individual modified histone moiety, or whether detected or measured as a histone component of a cell free mononucleosome, oligonucleosome or other chromatin fragment, for example; using a chromatographic, mass spectrophotometric, biosensor, chromatin immunoprecipitation (ChIP), immunoassay or other method of detection. In a preferred embodiment nucleosome associated modified histones are measured by means of a 2-site immunoassay (immunometric) method utilising one antibody or other selective binder to a nucleosome epitope and another to the particular histone modification of interest. We have shown that the relative levels of nucleosome associated histone post-translational modification levels are altered in the stool samples of diseased patients compared to the levels of stool samples of healthy subjects.

In one embodiment of the invention a group or class of related histone modifications (rather than a single modification) is investigated in a fecal sample. A typical example of this embodiment, without limitation, would involve a 2-site immunoassay employing one antibody or other selective binder directed to bind to nucleosomes and one antibody or other selective binder directed to bind the group of histone modifications in question. Examples of such antibodies directed to bind to a group of histone modifications would include, for illustrative purposes without limitation, anti-pan-acetylation antibodies (eg; a Pan-acetyl H4 antibody), anti-citrullination antibodies or anti-ubiquitination antibodies.

In an alternative embodiment, the epigenetic feature of a cell-free nucleosome comprises one or more histone variants or isoforms. Many histone variants are known in the art (for example; variants of histone H2 include H2A1, H2A2, mH2A1, mH2A2, H2AX and H2AZ), and this number is increasing with newly identified isoforms. Histone isoform variant protein structures are also amenable to post translational modification. For example; the H2A variant H2AX may be post translationally phosphorylated at serine 139 and this moiety is often called gamma-H2AX. Any histone variant, including any modified variant, may be a suitable feature for use in the invention whether detected or measured as individual histone variant moieties, for example using an immunoassay, chromatographic, mass spectrophotometric, ChIP, biosensor or other method for detection of a histone isoform moiety, or whether detected or measured as a histone component of a cell free mononucleosome, oligonucleosome or other chromatin fragment incorporating the histone variant. In a preferred embodiment nucleosome associated histone variants are measured by means of a 2-site immunoassay utilising one antibody or other selective binder to a nucleosome epitope and another to the particular histone variant or isoform of interest. We have shown that the relative levels of nucleosome associated histone variant levels are altered in the stool samples of diseased patients compared to the levels of stool samples of healthy subjects.

In an alternative embodiment, the epigenetic feature of a cell-free nucleosome comprises one or more DNA modifications. A number of particular DNA modifications are known in the art (for example; methylation, hydroxymethylation and carboxymethylation of cytosine) and this number is increasing with newly identified modifications. Any modified nucleotide or nucleoside or any DNA modification may be a suitable feature for use in the invention whether detected or measured in isolated DNA, nucleic acid, nucleotide or nucleoside moieties, for example using an immunoassay, chromatographic, mass spectrophotometric, ChIP, biosensor or other method for detection of a modified nucleic acid moiety, or whether detected or measured by any method for cell free mononucleosomes, oligonucleosomes or other chromatin fragments incorporating the particular DNA modification. In a preferred embodiment nucleosome associated DNA modifications are measured by means of a 2-site immunoassay utilising one antibody or other selective binder to a nucleosome epitope and another to the particular DNA modification of interest. We have shown that the relative levels of nucleosome associated 5-methylcytosine levels are altered in the stool samples of diseased patients compared to the levels of stool samples of healthy subjects. For clarity, the method of the present invention is not concerned with assessment of the methylation (or other DNA modification) status of any particular DNA sequence or gene, but with the global DNA modification status of the nucleosomes or other chromatin fragments present in the fecal test samples.

In an alternative embodiment, the epigenetic feature of a cell-free nucleosome comprises one or more protein-nucleosome adducts or complexes. A large number number of protein-nucleosome adducts or complexes are known to occur in chromatin (for example; HMGB, EZH2 and nuclear hormone receptor-nucleosome adducts) and this number is increasing with newly identified proteins that are associated with chromatin. Any protein-nucleosome adduct may be a suitable feature for use in the invention whether detected or measured by any method for cell free mononucleosome adducts, oligonucleosome adducts or other chromatin fragment adducts incorporating the particular protein, for example a 2-site immunoassay utilising one antibody or other selective binder to a nucleosome epitope and another to the particular protein comprised in the adduct. We have shown that the relative levels of nucleosome associated nucleosome adduct levels are altered in the stool samples of diseased patients compared to the levels of stool samples of healthy subjects.

In one embodiment, two or more measurements of cell-free nucleosomes *per se* and/or cell-free nucleosome epigenetic features are performed as a panel of nucleosome features.

In a further embodiment, the biomarker comprises a panel of biomarkers selected from: the level of nucleosomes, one or more particular histone modifications or cell free nucleosomes comprising a particular histone modification, histone variants or cell free nucleosomes comprising a particular histone variant, DNA modifications or cell free nucleosomes comprising a particular DNA modification or nucleosome adducts. As different populations may not be epigenetically identical or similar, the optimum panels selected for different animals or different populations may vary. Thus a panel of such biomarkers selected for use to detect CRC (for example) in humans may not be the same as a panel selected for use in another species (for example cats or dogs). Similarly the optimal panel for use to detect CRC in male and female humans (or any other animal) may be different. Similarly the optimal panel for use to detect CRC in different human sub-populations or races may differ. Alternatively, the same panel may be used in different populations (for example in males and females) but with different weightings used for the panel assay members. For example; the test value result of a three assay panel comprising assays "A", "B" and "C" may be calculated from a mathematical expression such as (Test Value = A + 2B + 3C) in males with a cut-off value of "X" and from a different mathematical expression such as (Test Value = 3A + 2B + C) in females with a different cut-off value of "Y".

According to a further aspect of the invention, there is a method for detecting or quantifying an epigenetic feature of a cell-free nucleosome in a fecal sample obtained from an animal or a human subject which comprises the steps of:(i) contacting the fecal sample with a first binding agent which binds to cell-free nucleosomes or a component thereof;(ii) contacting the fecal sample or cell-free nucleosomes with a second binding agent which binds to an epigenetic feature within said cell-free nucleosomes;(iii) detecting or quantifying the binding of said second binding agent to the epigenetic feature within said cell-free nucleosomes in the fecal sample; and(iv) using the presence, degree or amount of such binding as a measure of the presence of cell-free nucleosomes containing said epigenetic feature in the fecal sample.

The epigenetic feature referred to may be a particular post-translational histone modification, a particular histone variant or isoform, a particular DNA modification or a particular protein adducted to said nucleosome.

According to a further aspect of the invention, there is provided a method for detecting or quantifying an epigenetic feature of a cell-free nucleosome in a fecal sample obtained from an animal or a human subject which comprises the steps of:(i) contacting the fecal sample with a first binding agent which binds to an epigenetic feature within said cell-free nucleosomes;(ii) contacting the fecal sample or cell-free nucleosomes with a second binding agent which binds to cell-free nucleosomes or a component thereof;(iii) detecting or quantifying the binding of said second binding agent to cell-free nucleosomes or a component thereof in the fecal sample; and(iv) using the presence, degree or amount of such binding as a measure of the presence of cell-free nucleosomes containing said epigenetic feature in the fecal sample.

The epigenetic structure referred to may be a particular post-translational histone modification, a particular histone variant or isoform, a particular DNA modification or a particular protein adducted to said nucleosome.

In a further aspect of the invention the measured level of nucleosomes containing the particular epigenetic structure is used to determine the cellular origin of nucleosomes present in a fecal sample. For example; whether fecal nucleosomes originate from the chromatin of healthy gastroenterological cells, CRC cells, polyp cells or cells with other lesions or from any mixture of such cells. It will be clear to those skilled in the art that a subject whose colon or rectum contains cancer cells will also contain healthy cells and that a fecal sample obtained from such a subject may contain nucleosomes originating from healthy cells as well as from cancer cells and/or adenoma or polyp cells. Similarly nucleosomes with other combinations of origins may occur in subjects with different medical conditions including nucleosomes from healthy cells as well as nucleosomes from one or more types of diseased cells with one or more different lesions. In a preferred embodiment a panel of measured fecal nucleosome epigenetic structure levels is used to determine the cellular origin, or origins, of nucleosomes present in the sample.

According to a further aspect of the invention there is provided a method for determining the cell origin of fecal cell-free nucleosomes in an animal or a human subject which comprises the steps of:(i) detecting or measuring an epigenetic feature of a cell-free nucleosome in a fecal sample obtained from the subject; and(ii) using the measured level of cell-free nucleosomes containing said epigenetic feature as an indicator of whether the cell-free nucleosomes originate from healthy gastroenterological cells, colorectal cancer cells, polyp cells, cells with other lesions or any mixture thereof.

In a preferred embodiment of the invention several fecal assays of the invention (using any method of the invention) are performed to produce an epigenetic profile consisting of multiple epigenetic nucleosome features. The epigenetic profile may be used to determine the origin of nucleosomes present in the sample (whether originating from the chromatin of healthy gastroenterological cells, CRC cells, polyp cells or cells with other lesions or from any mixture of such cells). The epigenetic profile may be used to determine the presence or otherwise of a colorectal cancer, one or more polyps or other lesions in the subject from whom the fecal sample was taken.

According to a further aspect of the invention there is provided a method for detecting fecal cell-free nucleosomes with an epigenetic feature including nucleosome adducts and nucleosome associated histone modifications, DNA modifications or histone variants in a fecal sample obtained from a subject according to any of the above methods which comprises the steps of:
(i) obtaining a fecal sample from the subject;
(ii) contacting the fecal sample with a first and second binding agent wherein one of said binding agents binds to cell-free nucleosomes or a component thereof and the other of said binding agents binds to an epigenetic feature of said cell-free nucleosomes;
(iii) detecting or quantifying the binding of either or both of said binding agents in the fecal sample; and
(iv) using the presence, degree or amount of such binding as a measure of the presence of cell-free nucleosomes with said epigenetic feature in the fecal sample.

It will be appreciated that the epigenetic features described herein may also be assayed independently of their inclusion, or otherwise, within a nucleosome. Thus, according to a further aspect of the invention there is provided a method for diagnosing or detecting cancer, adenoma, autoimmune disease or inflammatory disease in an animal or a human subject which comprises the steps of:
(i) obtaining a fecal sample from the subject;
(ii) detecting or measuring the level of a particular histone variant, histone isoform or a histone containing a particular post-translational modification in the fecal sample; and
(iii) using the measured level of the particular histone variant, isoform or modification in the fecal sample as an indicator of the presence of said disease in the subject.

In one embodiment of the invention histones in a fecal sample are assayed to determine the histone variant, isoform or histone modification composition of the said sample. In one embodiment the histones in a fecal sample may be isolated for such an assay, for example without limitation, by acid extraction of the sample. In another embodiment there is provided an immunoassay for a histone modification employing a first anti-histone binding agent in combination with another binding agent directed to bind a histone modification on the same histone moiety. In another embodiment there is provided a histone variant or isoform immunoassay employing two anti-histone binding agents directed to bind different epitopes on the same histone moiety at least one of which is specific to the isoform of interest.

According to a further aspect of the invention there is provided a method for diagnosing or detecting colorectal cancer, a colorectal adenoma or a polyp in an animal or a human subject which comprises the steps of:(i) detecting or measuring an epigenetic feature of a cell-free nucleosome in a fecal sample obtained from the subject; and(ii) using the measured level of cell-free nucleosomes containing said epigenetic feature as indicative of the presence of said disease in the subject.

In a preferred embodiment of this aspect the epigenetically modified nucleotide measured in fecal chromatin fragments is 5-methylcytosine or methylated DNA. For clarity this aspect of the invention should not be confused with methods for gene methylation testing which involve the investigation of the cytosine methylation status of a particular gene or DNA sequence, for example as used in the Cologuard method. In contrast the present invention involves determination of the global level of 5-methylcytosine irrespective of gene sequence.

In one embodiment of the invention modified nucleotides in a fecal sample are assayed to determine the modified nucleotide composition of the said sample. In one embodiment the nucleotides and/or DNA in a fecal sample may be isolated for such an assay, for example without limitation, by organic solvent or chromatographic DNA extraction of the sample. In another embodiment there is provided an immunoassay for a modified nucleotide. This may be a single antibody immunoassay employing an anti-nucleotide binding agent, for example an anti-5methylcytosine binding agent, or a 2-site immunoassay, for example employing a first anti-single or double stranded DNA binding agent in combination with another binding agent directed to bind to a modified nucleotide.

In a preferred embodiment of the invention there is provided a 2-site immunoassay method for the measurement of nucleosome incorporated epigenetic features in situ employing an immobilized anti-nucleosome binding agent in combination with a labelled anti-histone modification or anti-histone variant or anti-DNA modification or anti-adducted protein detection binding agent. In another embodiment of the invention there is provided a 2-site immunoassay employing a labelled anti-nucleosome detection binding agent in combination with an immobilized anti-histone modification or anti-histone variant or anti-DNA modification or anti-adducted protein binding agent.

In one embodiment, the nucleosome adduct includes a High Mobility Group Protein, a polycomb protein, a chromatin modifying enzyme or a nuclear hormone receptor.

In one embodiment, the disease is selected from cancer, such as colorectal cancer, a colorectal adenoma or a polyp.

In a preferred embodiment a panel of multiple epigenetic nucleosome features is used to detect the presence of a colorectal cancer or a colorectal adenoma or polyp in the subject. In addition, further tests may be included within such a panel to increase its clinical accuracy including for example tests for haemoglobin, other known tumor markers including carcinoembryonic antigen (CEA) and/or specific mutated or methylated gene sequences. Additional parameters may include any relevant clinical information for example without limitation age, gender, Body Mass Index, smoking status and dietary habits.

In one embodiment of the invention the level of a particular structural epigenetic feature of a nucleosome in a fecal sample, or a panel of the levels of 2 or more such epigenetic features, is used to determine the optimal drug treatment or other treatment regime for a subject in need of such treatment.

Also disclosed is a method for assessment of an animal or a human subject for suitability for a medical treatment which comprises the steps of:
(i) detecting or measuring a cell-free nucleosome containing an epigenetic feature in a fecal sample obtained from said subject; and
(ii) using the measured level of cell-free nucleosomes with said feature detected as a parameter for selection of a suitable treatment for the subject.

According to a further aspect of the invention there is provided a method for monitoring the efficacy of a therapy in an animal or a human subject having, suspected of having, or of being predisposed to colorectal cancer, a colorectal adenoma or a polyp which comprises the steps of:(i) measuring an epigenetic feature of a cell-free nucleosome in a fecal sample obtained from the subject; and(ii) using the level of cell-free nucleosomes containing said epigenetic feature compared with an earlier sample taken from said subject as indicative of the efficacy of said therapy.

According to a further aspect of the invention there is provided a method for determining the prognosis of an animal or a human subject with colorectal cancer, a colorectal adenoma or a polyp which comprises the steps of:(i) measuring an epigenetic feature of a cell-free nucleosome in a fecal sample obtained from the subject; and(ii) using the level of cell-free nucleosomes containing said epigenetic feature as indicative of the prognosis of colorectal cancer, a colorectal adenoma or a polyp.

According to a further aspect of the disclosure there is provided a method for detecting or measuring fecal cell-free nucleosomes with an epigenetic feature, either alone or as part of a panel of measurements, for the purposes of detecting or diagnosing a disease status, or for assessment of an animal or a human subject for suitability for a medical treatment, or for monitoring a treatment of an animal or a human subject, for use in subjects with actual or suspected cancer, benign tumours, inflammatory disease or autoimmune disease.

Also disclosed is a method for identifying a fecal cell-free nucleosome associated epigenetic biomarker for detecting or diagnosing a disease status in an animal or a human subject which comprises the steps of:
(i) detecting or measuring a fecal cell-free nucleosome with an epigenetic feature in a fecal sample of a diseased subject;
(ii) detecting or measuring a cell-free nucleosome with the same epigenetic feature in a fecal sample of a healthy subject or a control subject; and
(iii) using the difference between the measured levels detected in a diseased and a control subject to identify whether a nucleosome with the epigenetic feature is useful as a biomarker for the disease status, either as a stand-alone biomarker or in combination with other biomarkers.

According to a further aspect of the invention there is provided the use of a kit comprising one or more binding agents capable of detecting and/or quantifying an epigenetic feature of a fecal cell-free nucleosome for the diagnosis or detection of colorectal cancer, a colorectal adenoma or a polyp in a fecal sample.

In one embodiment, the one or more binding agents comprises a ligand or binder specific for the cell-free nucleosome or component part thereof, or a structural/shape mimic of the DNA base, nucleotide or nucleoside or component part thereof.

According to a further aspect of the invention a panel of tests is provided to obtain an epigenetic profile of fecal nucleosomes with regard to multiple epigenetic nucleosome features. Such a test panel may consist, for example, of two or more measurements of nucleosomes containing different nucleosome epitopes; including without limitation different adducts and/or histone variants and/or histone modifications and/or DNA modifications and/or measurements of nucleosomes *per se,* or any combination or ratio of any of these and any other nucleosome epitopes. It will be clear to those skilled in the art that any of these panels may also include other (non-epigenetic and/or non nucleosomal) markers including haemoglobin and any other known tumor markers.

It will be appreciated that any of the aforementioned methods may be used either as a stand-alone test or in combination with existing tests.

The epigenetic nucleosome profile obtained may be used to determine the cells of origin of the chromatin fragments in which the nucleosomes were produced. Likely cellular origins may include for example healthy gastroenterological cells, inflammation cells, CRC cells or adenoma or polyp cells. The fecal chromatin fragments may include fragments originating from a mixture of two or more different cell types, for example without limitation from healthy cells as well as CRC or polyp cells. If any (even a small minority) of the nucleosome chromatin fragments present in the fecal sample have a CRC cell or polyp cell origin this clearly indicates the presence of a colorectal cancer or polyp in the subject from whom the fecal was obtained.

It will be clear to those skilled in the art that the two antibodies or other binders used may be directed to bind to intact nucleosomes or to any component part of a nucleosome including without limitation against a histone, a histone variant, a histone modification or any nucleotide (modified or otherwise) or other part of the DNA component of a nucleosome. This applies in any combination to both the immobilised and detection antibodies (or other binders) used in a classical 2-site immunoassay. Thus in a further aspect of the invention there is provided a method for detecting (only) those nucleosomes which contain both features to which the binders are directed. An advantage of this design is that the fecal nucleosome epitopes can be selected to be epitopes whose concurrence in fecal nucleosomes is associated with healthy gastroenterological cells or CRC cells or polyp cells or otherwise diseased cells. Use of such an antibody or binder selective for an epitope common to many or most or all nucleosomes of a disease (eg; cancer) origin, but which is absent or rarer in nucleosomes of a healthy cell origin, as an immunosorbent (usually solid phase antibody) will select for such nucleosomes of diseased cell origin. This provides a tumor nucleosome selectivity aspect to the assay.

In one embodiment of the invention, the antibody or other selective binder selected as the anti-nucleosome binder is selective for the enrichment of nucleosomes of tumor origin. In a preferred embodiment the antibody or other anti-nucleosome selective binder used is directed to bind to histone H3.1 and/or H3.2 and/or H3t. The binder selective for nucleosomes of tumor origin may be used in any assay including assays for the level of nucleosomes *per se* (of tumor origin), any particular histone modification or cell free nucleosomes comprising a particular histone modification, histone variant or cell free nucleosomes comprising a particular histone variant, DNA modification or cell free nucleosomes comprising a particular DNA modification or a nucleosome adduct.

Methods for the collection of stool or fecal samples have been in use for many years for FOBT and FIT testing and are well known in the art. One common method involves collection of fecal material by the patient and smearing a small amount of fecal material on a support which is then transported to a laboratory. In another method a small amount of fecal material collected by the patient is diluted into a buffer and transported to the laboratory. In some methods repeated collection, for example on 3 separate days, are made for multiple analysis to reduce the error involved in a single sample measurement, particularly for FOBT measurements.

Devices for the collection of fecal sample material are commercially available. In our studies fecal samples were collected using a collection device recommended for use with a commercially available FIT test. The device includes a sample stick which is used to scoop a small amount of stool material. The probe is then inserted into its tube, where excess sample is removed from the stick and 10mg of fecal sample is added to 2ml of buffer. The tube also contains a filter system to remove solids when a portion of the liquid sample is removed for analysis. It will be clear to those skilled in the art that any fecal sample collection method may be used for the invention. Similarly any fecal derived sample that has been diluted, filtered, separated, purified or otherwise prepared for analysis may be used be used for the invention.

Any method for isolating and/or detecting or measuring the level of histone modifications and/or cell free nucleosomes comprising a particular histone modification and/or histone variants and/or cell free nucleosomes comprising a particular histone variant and/or DNA modifications and/or cell free nucleosomes comprising a particular DNA modification and/or nucleosome adducts and/or nucleosomes *per se* as biomarkers in a fecal sample may be used for the invention. Examples of methods for the detection or measurement of theses analytes include chromatographic, spectroscopic methods (particularly mass spectrometry), biosensor, ChIP and immunochemical methods. Some detection or measurement methods may involve prior enrichment or isolation of these analytes from the fecal sample, for example by methods including chromatography or affinity purification/isolation using a selective antibody binder or other specific analyte binders. DNA modification isolation or purification may be achieved by DNA extraction methods known in the art.

It will be clear to those skilled in the art that the methods of the invention described include a variety of embodiments including biosensor type assays and label-free assays of the type marketed for example by ForteBio Incorporated of USA.

It will be clear to those skilled in the art that the terms antibody, binder or ligand in regard to any aspect of the invention is not limiting but intended to include any binder capable of binding to particular molecules or entities and that any suitable binder can be used in the method of the invention. It will also be clear that the term nucleosomes is intended to include mononucleosomes and oligonucleosomes and any such chromatin fragments that can be analysed in fluid media.

A further aspect of the disclosure provides ligands or binders, such as naturally occurring or chemically synthesised compounds, capable of specific binding to the biomarker. A ligand or binder according to the invention may comprise a peptide, an antibody or a fragment thereof, or a synthetic ligand such as a plastic antibody, or an aptamer or oligonucleotide, capable of specific binding to the biomarker. The antibody can be a monoclonal antibody or a fragment thereof capable of specific binding to the biomarker. A ligand according to the invention may be labeled with a detectable marker, such as a luminescent, fluorescent, enzyme or radioactive marker; alternatively or additionally a ligand according to the invention may be labelled with an affinity tag, e.g. a biotin, avidin, streptavidin or His (e.g. hexa-His) tag. Alternatively ligand binding may be determined using a label-free technology for example that of ForteBio Inc.

A biosensor according to the disclosure may comprise the biomarker or a structural/shape mimic thereof capable of specific binding to an antibody against the biomarker. Also provided is an array comprising a ligand or mimic as described herein.

Also provided by the disclosure is the use of one or more ligands as described herein, which may be naturally occurring or chemically synthesised, and is suitably a peptide, antibody or fragment thereof, aptamer or oligonucleotide, or the use of a biosensor of the invention, or an array of the invention, or a kit of the invention to detect and/or quantify the biomarker. In these uses, the detection and/or quantification can be performed on a biological sample as defined herein.

Diagnostic or monitoring kits are provided for performing methods of the invention. Such kits will suitably comprise a ligand according to the invention, for detection and/or quantification of the biomarker, and/or a biosensor, and/or an array as described herein, optionally together with instructions for use of the kit.

A further aspect of the disclosure is a kit for detecting the presence of a disease state, comprising a biosensor capable of detecting and/or quantifying one or more of the biomarkers as defined herein.

Biomarkers for detecting the presence of a disease are essential targets for discovery of novel targets and drug molecules that retard or halt progression of the disorder. As the level of the biomarker is indicative of disorder and of drug response, the biomarker is useful for identification of novel therapeutic compounds in *in vitro* and/or *in vivo* assays. Biomarkers of the disclosure can be employed in methods for screening for compounds that modulate the activity of the biomarker.

Thus, in a further aspect of the disclosure, there is provided the use of a binder or ligand, as described, which can be a peptide, antibody or fragment thereof or aptamer or oligonucleotide according to the invention; or the use of a biosensor according to the invention, or an array according to the invention; or a kit according to the invention, to identify a substance capable of promoting and/or of suppressing the generation of the biomarker.

Also there is provided a method of identifying a substance capable of promoting or suppressing the generation of the biomarker in a subject, comprising administering a test substance to a subject animal and detecting and/or quantifying the level of the biomarker present in a test sample from the subject.

The term "biomarker" means a distinctive biological or biologically derived indicator of a process, event, or condition. Biomarkers can be used in methods of diagnosis, e.g. clinical screening, and prognosis assessment and in monitoring the results of therapy, identifying subjects most likely to respond to a particular therapeutic treatment, drug screening and development. Biomarkers and uses thereof are valuable for identification of new drug treatments and for discovery of new targets for drug treatment.

The terms "detecting" and "diagnosing" as used herein encompass identification, confirmation, and/or characterisation of a disease state. Methods of detecting, monitoring and of diagnosis according to the invention are useful to confirm the existence of a disease, to monitor development of the disease by assessing onset and progression, or to assess amelioration or regression of the disease. Methods of detecting, monitoring and of diagnosis are also useful in methods for assessment of clinical screening, prognosis, choice of therapy, evaluation of therapeutic benefit, i.e. for drug screening and drug development.

Efficient diagnosis and monitoring methods provide very powerful "subject solutions" with the potential for improved prognosis, by establishing the correct diagnosis, allowing rapid identification of the most appropriate treatment (thus lessening unnecessary exposure to harmful drug side effects), and reducing relapse rates.

In one embodiment, said biomarker is released from the cells of a tumour. Thus, according to a further aspect of the invention there is provided a method for the detection of a tumour growth which comprises the steps of (i) measuring a biomarker in a biological sample that is associated with or released from the cells of a tumour and (ii) demonstrating that the level of said biomarker is associated with the size, stage, aggressiveness or dissemination of the tumour.

The immunoassays of the disclosure include any method employing one or more antibodies or other specific binders directed to bind to a nucleosome or to a nucleosome component or to an epigenetic feature of a nucleosome. Immunoassays include 2-site immunoassays or immunometric assays employing enzyme detection methods (for example ELISA), fluorescence labelled immunometric assays, time-resolved fluorescence labelled immunometric assays, chemiluminescent immunometric assays, immunoturbidimetric assays, particulate labelled immunometric assays and immunoradiometric assays as well as single-site immunoassays, reagent limited immunoassays, competitive immunoassay methods including labelled antigen and labelled antibody single antibody immunoassay methods with a variety of label types including radioactive, enzyme, fluorescent, time-resolved fluorescent and particulate labels. All of said immunoassay methods are well known in the art, see for example Salgame *et al,* 1997 and van Nieuwenhuijze *et al,* 2003.

In one embodiment, the method of the invention is repeated on multiple occasions. This embodiment provides the advantage of allowing the detection results to be monitored over a time period. Such an arrangement will provide the benefit of monitoring or assessing the efficacy of treatment of a disease state. Such monitoring methods of the invention can be used to monitor onset, progression, stabilisation, amelioration, relapse and/or remission.

Thus, the disclosure also provides a method of monitoring efficacy of a therapy for a disease state in a subject, suspected of having such a disease, comprising detecting and/or quantifying the biomarker present in a biological sample from said subject. In monitoring methods, test samples may be taken on two or more occasions. The method may further comprise comparing the level of the biomarker(s) present in the test sample with one or more control(s) and/or with one or more previous test sample(s) taken earlier from the same test subject, e.g. prior to commencement of therapy, and/or from the same test subject at an earlier stage of therapy. The method may comprise detecting a change in the nature or amount of the biomarker(s) in test samples taken on different occasions.

Thus, according to a further aspect of the disclosure, there is provided a method for monitoring efficacy of therapy for a disease state in a human or animal subject, comprising:
(i) quantifying the amount of the biomarker as defined herein; and
(ii) comparing the amount of said biomarker in a test sample with the amount present in one or more control(s) and/or one or more previous test sample(s) taken at an earlier time from the same test subject.

A change in the level of the biomarker in the test sample relative to the level in a previous test sample taken earlier from the same test subject may be indicative of a beneficial effect, e.g. stabilisation or improvement, of said therapy on the disorder or suspected disorder. Furthermore, once treatment has been completed, the method of the invention may be periodically repeated in order to monitor for the recurrence of a disease.

Methods for monitoring efficacy of a therapy can be used to monitor the therapeutic effectiveness of existing therapies and new therapies in human subjects and in non-human animals (e.g. in animal models). These monitoring methods can be incorporated into screens for new drug substances and combinations of substances. In a further embodiment the monitoring of more rapid changes due to fast acting therapies may be conducted at shorter intervals of hours or days.

According to a further aspect of the disclosure, there is provided a method for identifying a biomarker for detecting the presence of a disease state. The term "identifying" as used herein means confirming the presence of the biomarker present in the biological sample. Quantifying the amount of the biomarker present in a sample may include determining the concentration of the biomarker present in the sample. Identifying and/or quantifying may be performed directly on the sample, or indirectly on an extract therefrom, or on a dilution thereof.

In alternative aspects of the disclosure, the presence of the biomarker is assessed by detecting and/or quantifying antibody or fragments thereof capable of specific binding to the biomarker that are generated by the subject's body in response to the biomarker and thus are present in a biological sample from a subject having a disease state.

Identifying and/or quantifying can be performed by any method suitable to identify the presence and/or amount of a specific protein in a biological sample from a subject or a purification or extract of a biological sample or a dilution thereof. In methods of the invention, quantifying may be performed by measuring the concentration of the biomarker in the sample or samples. Biological samples that may be tested in a method of the invention include those as defined hereinbefore. The samples can be prepared, for example where appropriate diluted or concentrated, and stored in the usual manner.

Identification and/or quantification of biomarkers may be performed by detection of the biomarker or of a fragment thereof, e.g. a fragment with C-terminal truncation, or with N-terminal truncation. Fragments are suitably greater than 4 amino acids in length, for example 5, 6, 7, 8, 9, 10, 11, 12, 13, 14, 15, 16, 17, 18, 19, or 20 amino acids in length. It is noted in particular that peptides of the same or related sequence to that of histone tails are particularly useful fragments of histone proteins.

The biomarker may be directly detected, e.g. by SELDI or MALDI-TOF. Alternatively, the biomarker may be detected directly or indirectly via interaction with a ligand or ligands such as an antibody or a biomarker-binding fragment thereof, or other peptide, or ligand, e.g. aptamer, or oligonucleotide, capable of specifically binding the biomarker. The ligand or binder may possess a detectable label, such as a luminescent, fluorescent or radioactive label, and/or an affinity tag.

For example, detecting and/or quantifying can be performed by one or more method(s) selected from the group consisting of: SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC and other LC or LC MS-based techniques. Appropriate LC MS techniques include ICAT® (Applied Biosystems, CA, USA), or iTRAQ® (Applied Biosystems, CA, USA). Liquid chromatography (e.g. high pressure liquid chromatography (HPLC) or low pressure liquid chromatography (LPLC)), thin-layer chromatography, NMR (nuclear magnetic resonance) spectroscopy could also be used.

Methods of diagnosing or monitoring according to the invention may comprise analysing a sample by SELDI TOF or MALDI TOF to detect the presence or level of the biomarker. These methods are also suitable for clinical screening, prognosis, monitoring the results of therapy, identifying subjects most likely to respond to a particular therapeutic treatment, for drug screening and development, and identification of new targets for drug treatment.

Identifying and/or quantifying the analyte biomarkers may be performed using an immunological method, involving an antibody, or a fragment thereof capable of specific binding to the biomarker. Suitable immunological methods include 2-site immunoassays (immunometric assays or sandwich immunoassays), such as sandwich ELISA, in which the detection of the analyte biomarkers is performed using two antibodies which recognize different epitopes on a analyte biomarker; radioimmunoassays (RIA), direct, indirect or competitive enzyme linked immunosorbent assays (ELISA), enzyme immunoassays (EIA), Fluorescence immunoassays (FIA), western blotting, immunoprecipitation and any particle-based immunoassay (e.g. using gold, silver, or latex particles, magnetic particles, or Q-dots). Immunological methods may be performed, for example, in microtitre plate or strip format. In one embodiment, detection or measurement comprises an immunoassay, immunochemical, mass spectroscopy, chromatographic, chromatin immunoprecipitation or biosensor method.

According to a further aspect of the disclosure, there is provided a biomarker identified by the method as defined herein.

In one embodiment, one or more of the biomarkers may be replaced by a molecule, or a measurable fragment of the molecule, found upstream or downstream of the biomarker in a biological pathway.

According to a further aspect of the invention there is provided the use of a post-translational histone modification, a histone variant or isoform or a modified nucleotide as a biomarker in a fecal sample for the diagnosis or detection of cancer, adenoma, autoimmune disease or inflammatory disease.

The identification of key biomarkers specific to a disease is central to integration of diagnostic procedures and therapeutic regimes. Using predictive biomarkers appropriate diagnostic tools such as biosensors can be developed; accordingly, in methods and uses of the invention, identifying and quantifying can be performed using a biosensor, microanalytical system, microengineered system, microseparation system, immunochromatography system or other suitable analytical devices. The biosensor may incorporate an immunological method for detection of the biomarker(s), electrical, thermal, magnetic, optical (e.g. hologram) or acoustic technologies. Using such biosensors, it is possible to detect the target biomarker(s) at the anticipated concentrations found in biological samples.

As used herein, the term "biosensor" means anything capable of detecting the presence of the biomarker. Examples of biosensors are described herein.

Biosensors according to the disclosure may comprise a ligand binder or ligands, as described herein, capable of specific binding to the biomarker. Such biosensors are useful in detecting and/or quantifying a biomarker of the invention.

The biomarker(s) of the disclosure can be detected using a biosensor incorporating technologies based on "smart" holograms, or high frequency acoustic systems, such systems are particularly amenable to "bar code" or array configurations.

In smart hologram sensors (Smart Holograms Ltd, Cambridge, UK), a holographic image is stored in a thin polymer film that is sensitised to react specifically with the biomarker. On exposure, the biomarker reacts with the polymer leading to an alteration in the image displayed by the hologram. The test result read-out can be a change in the optical brightness, image, colour and/or position of the image. For qualitative and semi-quantitative applications, a sensor hologram can be read by eye, thus removing the need for detection equipment. A simple colour sensor can be used to read the signal when quantitative measurements are required. Opacity or colour of the sample does not interfere with operation of the sensor. The format of the sensor allows multiplexing for simultaneous detection of several substances. Reversible and irreversible sensors can be designed to meet different requirements, and continuous monitoring of a particular biomarker of interest is feasible.

Suitably, biosensors for detection of one or more biomarkers of the disclosure combine biomolecular recognition with appropriate means to convert detection of the presence, or quantitation, of the biomarker in the sample into a signal. Biosensors can be adapted for "alternate site" diagnostic testing, e.g. in the ward, outsubjects' department, surgery, home, field and workplace.

Biosensors to detect one or more biomarkers of the disclosure include acoustic, plasmon resonance, holographic, Bio-Layer Interferometry (BLI) and microengineered sensors. Imprinted recognition elements, thin film transistor technology, magnetic acoustic resonator devices and other novel acousto-electrical systems may be employed in biosensors for detection of the one or more biomarkers of the invention.

Methods involving identification and/or quantification of one or more biomarkers of the disclosure can be performed on bench-top instruments, or can be incorporated onto disposable, diagnostic or monitoring platforms that can be used in a non-laboratory environment, e.g. in the physician's office or at the subject's bedside. Suitable biosensors for performing methods of the invention include "credit" cards with optical or acoustic readers. Biosensors can be configured to allow the data collected to be electronically transmitted to the physician for interpretation and thus can form the basis for e-medicine.

Diagnostic kits for the diagnosis and monitoring of the presence of a disease state are described herein. In one embodiment, the kits additionally contain a biosensor capable of identifying and/or quantifying a biomarker. Suitably a kit according to the invention may contain one or more components selected from the group: a ligand binder, or ligands, specific for the biomarker or a structural/shape mimic of the biomarker, one or more controls, one or more reagents and one or more consumables; optionally together with instructions for use of the kit in accordance with any of the methods defined herein.

The identification of biomarkers for a disease state permits integration of diagnostic procedures and therapeutic regimes. Detection of a biomarker of the invention can be used to screen subjects prior to their participation in clinical trials. The biomarkers provide the means to indicate therapeutic response, failure to respond, unfavourable side-effect profile, degree of medication compliance and achievement of adequate serum drug levels. The biomarkers may be used to provide warning of adverse drug response. Biomarkers are useful in development of personalized therapies, as assessment of response can be used to fine-tune dosage, minimise the number of prescribed medications, reduce the delay in attaining effective therapy and avoid adverse drug reactions. Thus by monitoring a biomarker of the invention, subject care can be tailored precisely to match the needs determined by the disorder and the pharmacogenomic profile of the subject, the biomarker can thus be used to titrate the optimal dose, predict a positive therapeutic response and identify those subjects at high risk of severe side effects.

Biomarker-based tests provide a first line assessment of 'new' subjects, and provide objective measures for accurate and rapid diagnosis, not achievable using the current measures.

Furthermore, diagnostic biomarker tests are useful to identify family members or subjects with mild or asymptomatic disease or who may be at high risk of developing symptomatic disease. This permits initiation of appropriate therapy, or preventive measures, e.g. managing risk factors. These approaches are recognised to improve outcome and may prevent overt onset of the disorder.

Biomarker monitoring methods, biosensors and kits are also vital as subject monitoring tools, to enable the physician to determine whether relapse is due to worsening of the disorder. If pharmacological treatment is assessed to be inadequate, then therapy can be reinstated or increased; a change in therapy can be given if appropriate. As the biomarkers are sensitive to the state of the disorder, they provide an indication of the impact of drug therapy.

According to a further aspect of the disclosure there is provided a method of treating cancer, adenoma, autoimmune disease or inflammatory disease in an animal or a human subject, which comprises the following steps:
(i) detecting or measuring an epigenetic feature of a cell-free nucleosome in the fecal sample of the subject;
(ii) using the measured level of cell-free nucleosomes containing said epigenetic feature as indicative of the presence of said disease in the subject; and
(iii) administering a therapeutic agent to a subject diagnosed in step (ii) as a patient with cancer, adenoma, autoimmune disease or inflammatory disease.

According to a further aspect of the disclosure there is provided a method of treating cancer, adenoma, autoimmune disease or inflammatory disease in an individual in need thereof, which comprises the step of administering a therapeutic agent to a patient identified as having differing levels of an epigenetic feature of a cell-free nucleosome in a fecal sample when compared to the levels of said epigenetic feature of a cell-free nucleosome from a control subject.

In one embodiment, the method of treatment comprises treatment of cancer, such as colorectal cancer, a colorectal adenoma or a polyp.

The invention will now be illustrated with reference to the following non-limiting examples.

### EXAMPLE 1

Fecal samples are collected from 150 subjects who are subsequently examined by colonoscopy following a positive FIT test in a routine CRC screening programme. Samples are collected using a sample probe to scoop a small amount of fecal material. The probe is then inserted into its tube, where excess sample is removed from the probe and 10mg of fecal sample is added to 2ml of buffer. Each tube also contains a filter system to remove solids from the liquid sample before analysis. The diluted fecal samples are tested using methods of the present invention for multiple different nucleosome assays including nucleosome concentration *per se,* as well as a number of nucleosome associated epigenetic features including one or more DNA modifications including 5-methylcytosine, one or more histone variants including gamma-H2AX and H2AZ, one or more histone modifications including H3K9Me3, H3K9Ac, H3K27Me3, H4K16Ac, H4K20Me3, ubiquityl-H2A and H4PanAc (pan-acetylatedH4). For each assay, 50 µL of diluted fecal material is added in duplicate to a microtitre well pre-coated with an anti-nucleosome antibody in duplicate and incubated overnight at 4°C. The wells are washed three times with a wash buffer (200 µL/well, 0.05M TRIS/HCI buffer pH 7.5 containing 1% Tween 20) and 50 µL of a biotinylated antibody solution (diluted in 0.05M TRIS/HCI pH 7.5 containing 0.9% NaCl, 0.05% sodium deoxycholate and 1% Nonidet P40 substitute) directed to bind to the epigenetic structure of interest (i.e. directed to bind to nucleosomes *per se,* or 5-methylcytosine, or gamma-H2AX, or H2AZ, or H3K9Me3, or H3K9Ac, or H3K27Me3, or H4K16Ac, or H4K20Me3, or ubiquityl-H2A or H4PanAc respectively). The wells were incubated for a further 90 minutes at room temperature, washed again as before and horse radish peroxidase enzyme-labelled streptavidin solution was added and incubated for a further 30 minutes. The wells were washed again and a coloured substrate solution (100 µL/well, 2,2'-Azinobis [3-ethylbenzothiazoline-6-sulfonic acid]-diammonium salt) was added and incubated for 20 minutes at room temperature with mild agitation. The optical density (OD) of the wells was measured at a wavelength of 405nm using a standard microtitre plate reader. A low background OD signal was observed in the negative control containing no nucleosomes and higher OD signals were observed for subject fecal samples. This demonstrates the presence of nucleosomes in the fecal samples.

### EXAMPLE 2

The OD results of each of the individual fecal epigenetic nucleosome assays described in EXAMPLE 1 are plotted as a dot plot and as a Receiver Operator Characteristic (ROC) curve. The ROC curves, and the areas under the ROC curves, show that some of the epigenetic features tested are more altered in CRC or polyp cells compared to healthy cells than others. Moreover when these assays are combined for use as a panel; highly accurate tests are obtained showing highly sensitive and specific discrimination of cancer from healthy patients for several different assay combinations. Combining individual assay results into a panel test is done using any multi-parameter analysis method of which many are known in the art. Regression analysis (for example, Fisher's linear discriminant analysis) is one common method. These fecal panel tests are safer and lower cost than colonoscopy methods for cancer detection and the clinical accuracy achieved exceeds that of FOBT and FIT tests.

### EXAMPLE 3

A fecal sample is obtained from a CRC patient prior to a possible treatment regime with a drug (for example with an HDACi, HMTi, DNMTi or other epigenetic drug) and the epigenetic status of the tumor is assessed using a method of the invention (for example a 2-site immunoassay for nucleosomes containing a particular epigenetic feature or a panel of such 2-site immunoassays). The epigenetic status of fecal nucleosomes is used to determine which treatment regime is likely to be an effective therapy for the patient.

### EXAMPLE 4

A fecal sample is obtained from a CRC patient prior to a treatment regime with a drug (for example an HDACi, HMTi, DNMTi or other epigenetic drug) and the epigenetic status of the tumor is assessed using a method of the invention (for example a 2-site immunoassay for nucleosomes containing a particular epigenetic feature or a panel of such 2-site immunoassays). Further fecal samples are obtained from the patient following treatment and the epigenetic status of fecal nucleosomes is reassessed to check or monitor the patient for the efficacy of the treatment.

### EXAMPLE 5

A fecal sample is obtained from a CRC patient and the epigenetic status of the tumor is assessed using a method of the invention (for example a 2-site immunoassay for nucleosomes containing a particular epigenetic feature or a panel of such 2-site immunoassays). The epigenetic status of fecal nucleosomes is used to assess the prognosis of for the patient.

### EXAMPLE 6

A fecal sample was collected from each of 3 subjects to test for the presence of nucleosomes in the sample using a commercially available fecal sample collection system as described in EXAMPLE 1. A sample probe to scoop a small amount of fecal material. The probe was then inserted into its tube, where excess sample was removed from the probe and 10mg of the fecal sample was added to 2ml of buffer. The tube also contained a filter system to remove solids from the liquid sample before analysis.

The diluted fecal samples were tested for cell free nucleosome content in duplicate by an ELISA method of the invention as follows. An anti-histone antibody coated microtitre plate was prepared by adding 100 µL of an anti-nucleosome antibody solution (15ug/ml in 0.1M phosphate buffer pH7.4) to each microtitre well and the wells were incubated for 9 hours at 4°C. The antibody solution was then removed and wells were blocked by addition of 200 µL of a 0.1M phosphate buffer pH7.4 solution containing 5% sucrose and 0.1% Tween 20 and the wells were incubated for 30 minutes at room temperature. The blocking solution was removed and the wells were washed 3 times with 200 µL of wash buffer (as described in EXAMPLE 1). To each well was added either 100 µL of diluted fecal sample solution or 10 µL of diluted fecal sample solution and 50 µL of assay buffer. The wells were incubated for 2.5 hours at room temperature with mild agitation. The diluted sample was then removed and the wells washed a further 3 times with 200 µL of wash buffer. Biotinylated anti-histone antibody diluted in 50 µL of assay buffer was then added and incubated for 1.5 hours at room temperature with mild agitation. The biotinylated antibody solution was then removed and the wells washed a further 3 times with 200 µL of wash buffer. A streptavidin-horse radish peroxidase conjugate solution in 50 µL of assay buffer was then added and incubated for 0.5 hours at room temperature with mild agitation. The streptavidin-horse radish peroxidase conjugate solution was then removed and the wells washed a further 3 times with 200 µL of wash buffer. A coloured substrate solution (100 µL/well, 2,2'-Azinobis [3-ethylbenzothiazoline-6-sulfonic acid]-diammonium salt) was added and incubated for 20 minutes at room temperature with mild agitation. The optical density (OD) of the wells was measured at a wavelength of 405nm using a standard microtitre plate reader.

The results are shown in Figure 1 with error bars indicating the reproducibility of the duplicate experimental results. A low OD signal was observed for sample 1, both for 10 µL/well and 100 µL/well, indicating that the level of cell free nucleosomes in this sample was low or undetectable. For sample 2 a higher OD signal was observed for 100 µL/well indicating that 100 µL of this sample contained a detectable level of cell free nucleosomes. However, the nucleosomes present in 10 µL were not detectable. For sample 3 a higher OD signal was observed both for 10 µL/well and 100 µL/well indicating that a higher level of cell free nucleosomes in this sample than in sample 1 or sample 2.

The same anti-histone antibody was used in this experiment as both the coated antibody and the biotinylated antibody. As the same epitope on a single histone molecule cannot be bound twice, a positive signal in the assay indicates the presence of intact nucleosomes in the fecal sample. To our knowledge, this is the first demonstration of the presence of cell free nucleosomes in a fecal sample and also the first demonstration of a method for the measurement or detection of cell free nucleosomes in a fecal sample.

### REFERENCES

Allen et al, A simple method for estimating global DNA methylation using bisulfite PCR of repetitive DNA elements. Nucleic Acids Research: 32(3) e38DOI: 10.1093/nar/gnh032
Esteller, Cancer epigenomics: DNA methylomes and histone-modification maps Nature Reviews Genetics: 8, 286-298, 2007
Feinberg and Vogelstein, Hypomethylation distinguishes genes of some human cancers from their normal counterparts. Nature: 301, 89-92, 1983
Grutzmann et al, Sensitive Detection of Colorectal Cancer in Peripheral Blood by Septin 9 DNA Methylation Assay. PLoS ONE 3(11): e3759. doi:10.1371/journal.pone.0003759, 2008
Hervouet et al, Disruption of Dnmt1/PCNA/UHRF1 Interactions Promotes Tumorigenesis from Human and Mice Glial Cells PLoS ONE 5(6): e11333. doi:10.1371/journal.pone.0011333, 2010
Herranz and Esteller, DNA methylation and histone modifications in patients with cancer: potential prognostic and therapeutic targets. Methods Mol Biol.361:25-62, 2007
Holdenrieder et al, Nucleosomes in serum of patients with benign and malignant diseases. Int. J. Cancer (Pred. Oncol.): 95, 114-120, 2001 Jiang et al, Blood. 102, 2243-50, 2003
The Medical Letter on Drugs and Therapeutics. 56, 100-101, 2014
Rodriguez-Paredes and Esteller, Cancer epigenetics reaches mainstream oncology. Nature Medicine: 17(3), 330-339, 2011
Salgame et al, An ELISA for detection of apoptosis. Nucleic Acids Research, 25(3), 680-681, 1997
van Nieuwenhuijze et al, Time between onset of apoptosis and release of nucleosomes from apoptotic cells: putative implications for systemic lupus erythematosus. Ann Rheum Dis; 62: 10-14, 2003
Yoshida and Shimura, Isolation of nonhistone chromosomal protein from calf thymus. Biochimica et Biophysica Acta (BBA) - Protein Structure; 263(3), 690-695, 1972

## Claims

1. Use of a cell-free nucleosome as a biomarker in a fecal sample for the diagnosis or detection of colorectal cancer, a colorectal adenoma or a polyp.

2. Use as defined in claim 1, wherein the biomarker is an epigenetic feature of a cell-free nucleosome.

3. Use as defined in claim 1 or claim 2, wherein the cell-free nucleosome is a mononucleosome or oligonucleosome.

4. A method for diagnosing or detecting colorectal cancer, a colorectal adenoma or a polyp in an animal or a human subject which comprises the steps of:
(i) detecting or measuring an epigenetic feature of a cell-free nucleosome in a fecal sample obtained from the subject; and
(ii) using the measured level of cell-free nucleosomes containing said epigenetic feature as indicative of the presence of said disease in the subject.

5. The use or method as defined in any one of claims 2 to 4, wherein the epigenetic feature is selected from: a post-translational histone modification, a histone variant or isoform, a DNA modification or a protein adducted to the nucleosome, such as a DNA modification selected from 5-methylcytosine, one or more histone variants selected from gamma-H2AX and H2AZ or one or more histone modifications selected from: H3K9Me3, H3K9Ac, H3K27Me3, H4K16Ac, H4K20Me3, ubiquityl-H2A and H4PanAc (pan-acetylatedH4).

6. A method for monitoring the efficacy of a therapy in an animal or a human subject having, suspected of having, or of being predisposed to colorectal cancer, a colorectal adenoma or a polyp which comprises the steps of:
(i) measuring an epigenetic feature of a cell-free nucleosome in a fecal sample obtained from the subject; and
(ii) using the level of cell-free nucleosomes containing said epigenetic feature compared with an earlier sample taken from said subject as indicative of the efficacy of said therapy.

7. A method for determining the prognosis of an animal or a human subject with colorectal cancer, a colorectal adenoma or a polyp which comprises the steps of:
(i) measuring an epigenetic feature of a cell-free nucleosome in a fecal sample obtained from the subject; and
(ii) using the level of cell-free nucleosomes containing said epigenetic feature as indicative of the prognosis of colorectal cancer, a colorectal adenoma or a polyp.

8. A method for determining the cell origin of fecal cell-free nucleosomes in an animal or a human subject which comprises the steps of:
(i) detecting or measuring an epigenetic feature of a cell-free nucleosome in a fecal sample obtained from the subject; and
(ii) using the measured level of cell-free nucleosomes containing said epigenetic feature as an indicator of whether the cell-free nucleosomes originate from healthy gastroenterological cells, colorectal cancer cells, polyp cells, cells with other lesions or any mixture thereof.

9. The use or method as defined in any one of claims 1 to 8, wherein said detection or measurement comprises an immunoassay, immunochemical, mass spectroscopy, chromatographic, chromatin immunoprecipitation or biosensor method.

10. The use or method as defined in any one of claims 1 to 9, wherein two or more measurements of cell-free nucleosomes *per se* and/or cell-free nucleosome epigenetic features are performed as a panel of nucleosome features.

11. Use of a kit comprising one or more binding agents capable of detecting and/or quantifying an epigenetic feature of a fecal cell-free nucleosome for the diagnosis or detection of colorectal cancer, a colorectal adenoma or a polyp in a fecal sample.

12. A method for detecting or quantifying an epigenetic feature of a cell-free nucleosome in a fecal sample obtained from an animal or a human subject which comprises the steps of:
(i) contacting the fecal sample with a first binding agent which binds to cell-free nucleosomes or a component thereof;
(ii) contacting the fecal sample or cell-free nucleosomes with a second binding agent which binds to an epigenetic feature within said cell-free nucleosomes;
(iii) detecting or quantifying the binding of said second binding agent to the epigenetic feature within said cell-free nucleosomes in the fecal sample; and
(iv) using the presence, degree or amount of such binding as a measure of the presence of cell-free nucleosomes containing said epigenetic feature in the fecal sample.

13. A method for detecting or quantifying an epigenetic feature of a cell-free nucleosome in a fecal sample obtained from an animal or a human subject which comprises the steps of:
(i) contacting the fecal sample with a first binding agent which binds to an epigenetic feature within said cell-free nucleosomes;
(ii) contacting the fecal sample or cell-free nucleosomes with a second binding agent which binds to cell-free nucleosomes or a component thereof;
(iii) detecting or quantifying the binding of said second binding agent to cell-free nucleosomes or a component thereof in the fecal sample; and
(iv) using the presence, degree or amount of such binding as a measure of the presence of cell-free nucleosomes containing said epigenetic feature in the fecal sample.

14. The method as defined in claim 12 or claim 13, wherein the epigenetic feature is selected from: a post-translational histone modification, a histone variant or isoform, a DNA modification or a protein adducted to said nucleosome, such as a DNA modifications selected from 5-methylcytosine, one or more histone variants selected from gamma-H2AX and H2AZ or one or more histone modifications selected from: H3K9Me3, H3K9Ac, H3K27Me3, H4K16Ac, H4K20Me3, ubiquityl-H2A and H4PanAc (pan-acetylatedH4).

## Patentansprüche

1. Verwendung eines zellfreien Nukleosoms als Biomarker in einer Fäkalprobe zur Diagnose oder Erfassung von Darmkrebs, einem Darmadenom oder einem Polypen.

2. Verwendung nach Anspruch 1, wobei der Biomarker ein epigenetisches Merkmal eines zellfreien Nukleosoms ist.

3. Verwendung nach Anspruch 1 oder Anspruch 2, wobei das zellfreie Nukleosom ein Mononukleosom oder ein Oligonukleosom ist.

4. Verfahren zur Diagnose oder Erfassung von Darmkrebs, einem Darmadenom oder einem Polypen bei einem tierischen oder einem menschlichen Subjekt, das die folgenden Schritte umfasst:
(i) Erfassen oder Messen eines epigenetischen Merkmals eines zellfreien Nukleosoms in einer Fäkalprobe, die von dem Subjekt erhalten wird; und
(ii) Verwenden des gemessenen Maßes an zellfreien Nukleosomen, die das epigenetische Merkmal enthalten, als das Vorhandensein der Erkrankung bei dem Subjekt angebend.

5. Verwendung oder Verfahren nach einem der Ansprüche 2 bis 4, wobei das epigenetische Merkmal aus Folgendem ausgewählt ist: einer post-translationalen Histon-Modifikation, einer Histon-Variante oder -Isoform, einer DNA-Modifikation oder einem Protein adduziert zu dem Nukleosom, wie zum Beispiel einer DNA-Modifikation, die aus 5-Methylcytosin ausgewählt ist, einer oder mehreren Histon-Varianten, die aus gamma-H2AX und -H2AZ ausgewählt sind, oder einer oder mehreren Histon-Modifikationen, die aus Folgendem ausgewählt sind: H3K9Me3, H3K9Ac, H3K27Me3, H4K16Ac, H4K20Me3, Ubiquityl-H2A und H4PanAc (pan-acetyliertem H4).

6. Verfahren zum Überwachen der Wirksamkeit einer Therapie bei einem tierischen oder einem menschlichen Subjekt, das an Darmkrebs, einem Darmadenom oder einem Polypen leidet, im Verdacht steht, darunter zu leiden oder dafür prädisponiert ist, das die folgenden Schritte umfasst:
(i) Messen eines epigenetischen Merkmals eines zellfreien Nukleosoms in einer Fäkalprobe, die von dem Subjekt erhalten wird; und
(ii) Verwenden des Maßes an zellfreien Nukleosomen, die das epigenetische Merkmal enthalten, verglichen mit einer früheren Probe, die von dem Subjekt genommen wurde, als die Wirksamkeit der Therapie angebend.

7. Verfahren zum Bestimmen der Prognose eines tierischen oder eines menschlichen Subjekts mit Darmkrebs, einem Darmadenom oder einem Polypen, das die folgenden Schritte umfasst:
(i) Messen eines epigenetischen Merkmals eines zellfreien Nukleosoms in einer Fäkalprobe, die von dem Subjekt erhalten wird; und
(ii) Verwenden des Maßes an zellfreien Nukleosomen, die das epigenetische Merkmal enthalten, als die Prognose von Darmkrebs, einem Darmadenom oder einem Polypen angebend.

8. Verfahren zum Bestimmen des Zellursprungs von fäkalen zellfreien Nukleosomen bei einem tierischen oder einem menschlichen Subjekt, das die folgenden Schritte umfasst:
(i) Erfassen oder Messen eines epigenetischen Merkmals eines zellfreien Nukleosoms in einer Fäkalprobe, die von dem Subjekt erhalten wird; und
(ii) Verwenden des gemessenen Maßes an zellfreien Nukleosomen, die das epigenetische Merkmal enthalten, als einen Indikator, ob die zellfreien Nukleosome von gesunden gastroenterologischen Zellen, Darmkrebszellen, Polypzellen, Zellen mit anderen Läsionen oder einer beliebigen Mischung davon stammen.

9. Verwendung oder Verfahren nach einem der Ansprüche 1 bis 8, wobei das Erfassen oder Messen einen Immunassay, ein immunochemisches Verfahren, Massenspektroskopie, ein chromatographisches Verfahren, Chromatin-Immunpräzipitation oder ein Biosensorverfahren umfasst.

10. Verwendung oder Verfahren nach einem der Ansprüche 1 bis 9, wobei zwei oder mehr Messungen an zellfreien Nukleosomen *per se* und/oder epigenetische Merkmale von zellfreien Nukleosomen als Paneel an Nukleosom-Merkmalen durchgeführt werden.

11. Verwendung eines Kits, das ein oder mehrere Bindemittel umfasst, die dazu in der Lage sind, ein epigenetisches Merkmal eines fäkalen zellfreien Nukleosoms zur Diagnose oder Erfassung von Darmkrebs, einem Darmadenom oder einem Polypen in einer Fäkalprobe zu erfassen und/oder zu quantifizieren.

12. Verfahren zum Erfassen oder Quantifizieren eines epigenetischen Merkmals eines zellfreien Nukleosoms in einer Fäkalprobe, die von einem tierischen oder einem menschlichen Subjekt erhalten wird, das die folgenden Schritte umfasst:
(i) Kontaktieren der Fäkalprobe mit einem ersten Bindemittel, das an zellfreie Nukleosome oder eine Komponente davon bindet;
(ii) Kontaktieren der Fäkalprobe oder der zellfreien Nukleosome mit einem zweiten Bindemittel, das an ein epigenetisches Merkmal innerhalb der zellfreien Nukleosome bindet;
(iii) Erfassen oder Quantifizieren der Bindung des zweiten Bindemittels an das epigenetische Merkmal innerhalb der zellfreien Nukleosome in der Fäkalprobe; und
(iv) Verwenden des Vorhandenseins, des Grads oder der Menge einer solchen Bindung als Maß des Vorhandenseins von zellfreien Nukleosomen, die das epigenetische Merkmal in der Fäkalprobe enthalten.

13. Verfahren zum Erfassen oder Quantifizieren eines epigenetischen Merkmals eines zellfreien Nukleosoms in einer Fäkalprobe, die von einem tierischen oder einem menschlichen Subjekt erhalten wird, das die folgenden Schritte umfasst:
(i) Kontaktieren der Fäkalprobe mit einem ersten Bindemittel, das an ein epigenetisches Merkmal innerhalb der zellfreien Nukleosome bindet;
(ii) Kontaktieren der Fäkalprobe oder der zellfreien Nukleosome mit einem zweiten Bindemittel, das an zellfreie Nukleosome oder eine Komponente davon bindet;
(iii) Erfassen oder Quantifizieren der Bindung des zweiten Bindemittels an zellfreie Nukleosome oder eine Komponente davon in der Fäkalprobe; und
(iv) Verwenden des Vorhandenseins, des Grads oder der Menge einer solchen Bindung als Maß des Vorhandenseins von zellfreien Nukleosomen, die das epigenetische Merkmal in der Fäkalprobe enthalten.

14. Verfahren nach Anspruch 12 oder Anspruch 13, wobei das epigenetische Merkmal aus Folgendem ausgewählt ist: einer post-translationalen Histon-Modifikation, einer Histon-Variante oder -Isoform, einer DNA-Modifikation oder einem Protein adduziert zu dem Nukleosom, wie zum Beispiel einer DNA-Modifikation, die aus 5-Methylcytosin ausgewählt ist, einer oder mehreren Histon-Varianten, die aus gamma-H2AX und - H2AZ ausgewählt sind, oder einer oder mehreren Histon-Modifikationen, die aus Folgendem ausgewählt sind: H3K9Me3, H3K9Ac, H3K27Me3, H4K16Ac, H4K20Me3, Ubiquityl-H2A und H4PanAc (pan-acetyliertem H4).

## Revendications

1. Utilisation d'un nucléosome acellulaire en tant que biomarqueur dans un échantillon de matière fécale pour le diagnostic ou la détection d'un cancer colorectal, d'un adénome colorectale ou d'un polype.

2. Utilisation selon la revendication 1, dans laquelle le biomarqueur est une caractéristique épigénétique d'un nucléosome acellulaire.

3. Utilisation selon la revendication 1 ou la revendication 2, dans laquelle le nucléosome acellulaire est un mononucléosome ou un oligonucléosome.

4. Procédé de diagnostic ou de détection d'un cancer colorectal, d'un adénome colorectal ou d'un polype chez un animal ou un sujet humain qui comprend les étapes suivantes :
(i) détection ou mesure d'une caractéristique épigénétique d'un nucléosome acellulaire dans un échantillon de matière fécale obtenu du sujet ; et
(ii) utilisation du niveau mesuré de nucléosomes acellulaires contenant ladite caractéristique épigénétique comme indiquant la présence de ladite maladie chez le sujet.

5. Utilisation ou procédé selon l'une quelconque des revendications 2 à 4, dans laquelle la caractéristique épigénétique est sélectionné parmi : une modification post-traductionnelle des histones, une variante ou un isoforme d'histone, une modification d'ADN ou une protéine ajoutée au nucléosome, par exemple une modification de l'ADN sélectionnée parmi la 5-méthylcytosine, une ou plusieurs variantes d'histones sélectionnées parmi les gamma-H2AX et H2AZ ou une ou plusieurs modifications d'histones sélectionnées parmi : H3K9Me3, H3K9Ac, H3K27Me3, H4K16Ac, H4K20Me3, ubiquityl-H2A et H4PanAc (H4 panacétylaté).

6. Procédé de surveillance de l'efficacité d'une thérapie chez un sujet animal ou humain, présentant, suspecté de présenter, ou prédisposé à un cancer colorectal, un adénome colorectal ou un polype qui comprend les étapes suivantes :
(i) mesure d'une caractéristique épigénétique d'un nucléosome acellulaire dans un échantillon de matière fécale obtenu du sujet ; et
(ii) utilisation du niveau des nucléosomes acellulaires contenant ladite caractéristique épigénétique comparé à un échantillon précédent prélevé sur ledit sujet comme indiquant l'efficacité de ladite thérapie.

7. Procédé de détermination du pronostic d'un sujet animal ou humain présentant un cancer colorectal, un adénome colorectal ou un polype qui comprend les étapes suivantes :
(i) mesure d'une caractéristique épigénétique d'un nucléosome acellulaire dans un échantillon de matière fécale obtenu du sujet ; et
(ii) utilisation du niveau de nucléosomes acellulaires contenant ladite caractéristique épigénétique comme indiquant le pronostic d'un cancer colorectal, d'un adénome colorectal ou d'un polype.

8. Procédé de détermination de l'origine cellulaire des nucléosomes acellulaires de matière fécale chez un sujet animal ou humain qui comprend les étapes suivantes :
(i) détection ou mesure d'une caractéristique épigénétique d'un nucléosome acellulaire dans un échantillon de matière fécale obtenu du sujet ; et
(ii) utilisation du niveau mesuré de nucléosomes acellulaires contenant ladite caractéristique épigénétique en tant qu'indicateur du fait que les nucléosomes acellulaires proviennent de cellules gastroentérologiques saines, de cellules de cancer colorectal, de cellules de polype, de cellules avec d'autres lésions ou de tout mélange de celles-ci.

9. Utilisation ou procédé selon l'une quelconque des revendications 1 à 8, dans lequel ladite détection ou mesure comprend un procédé de dosage immunologique, immunochimique, de spectrométrie de masse, chromatographique, d'immuno-précipitation de la chromatine ou de biocapteur.

10. Utilisation ou procédé selon l'une quelconque des revendications 1 à 9, dans lequel deux mesures ou plus des nucléosomes acellulaires en soi et/ou des caractéristiques épigénétiques des nucléosomes acellulaires sont réalisées comme un panel de caractéristiques de nucléosomes.

11. Utilisation d'un kit comprenant un ou plusieurs agents de liaison capables de détecter et/ou de quantifier une caractéristique épigénétique d'un nucléosome acellulaire de matière fécale pour le diagnostic ou la détection d'un cancer colorectal, d'un adénome colorectal ou d'un polype dans un échantillon de matière fécale.

12. Procédé de détection ou de quantification d'une caractéristique épigénétique d'un nucléosome acellulaire dans un échantillon de matière fécale obtenu du sujet animal ou humain qui comprend les étapes suivantes :
(i) mise en contact de l'échantillon de matière fécale avec un premier agent de liaison qui se lie aux nucléosomes acellulaires ou à un composant de ceux-ci ;
(ii) mise en contact de l'échantillon de matière fécale ou des nucléosomes acellulaires avec un second agent de liaison qui se lie à une caractéristique épigénétique à l'intérieur desdits nucléosomes acellulaires ;
(iii) détection ou quantification de la liaison dudit second agent de liaison à la caractéristique épigénétique à l'intérieur desdits nucléosomes acellulaires dans l'échantillon de matière fécale ; et
(iv) utilisation de la présence, du degré ou de la quantité de ces liaisons en tant que mesure de la présence de nucléosomes acellulaires contenant ladite caractéristique épigénétique dans l'échantillon de matière fécale.

13. Procédé de détection ou de quantification d'une caractéristique épigénétique d'un nucléosome acellulaire dans un échantillon de matière fécale obtenu du sujet animal ou humain qui comprend les étapes suivantes :
(i) mise en contact de l'échantillon de matière fécale avec un premier agent de liaison qui se lie à une caractéristique épigénétique à l'intérieur desdits nucléosomes acellulaires ;
(ii) mise en contact de l'échantillon de matière fécale ou des nucléoseomes acellulaires avec un second agent de liaison qui se lie à des nucléosomes acellulaires ou à un composant de ceux-ci ;
(iii) détection ou quantification de la liaison dudit second agent de liaison à des nucléosomes acellulaires ou un composant de ceux-ci dans l'échantillon de matière fécale ; et
(iv) utilisation de la présence, du degré ou de la quantité de ces liaisons en tant que mesure de la présence de nucléosomes acellulaires contenant ladite caractéristique épigénétique dans l'échantillon de matière fécale.

14. Procédé selon la revendication 12 ou la revendication 13, dans lequel la caractéristique épigénétique est sélectionné parmi : une modification post-traductionnelle des histones, une variante ou un isoforme d'histone, une modification d'ADN ou une protéine ajoutée au nucléosome, par exemple une modification de l'ADN sélectionnée parmi la 5-méthylcytosine, une ou plusieurs variantes d'histones sélectionnées parmi les gamma-H2AX et H2AZ ou une ou plusieurs modifications d'histones sélectionnées parmi : H3K9Me3, H3K9Ac, H3K27Me3, H4K16Ac, H4K20Me3, ubiquityl-H2A et H4PanAc (H4 pan-acétylaté).
